# EUROPÄISCHE PATENTANMELDUNG

(11) **EP 0 683 229 A2**
(43) Veröffentlichungstag der Anmeldung: **22.11.1995**
(21) Anmeldenummer: 95106801.4
(22) Anmeldetag: 05.05.1995
(51) Int. Cl.: C12N 15/63

(54) **Verfahren zur Herstellung von rekombinanten Aprotinin und rekombinanten Aprotinin Varianten mit der natürlichen N-terminalen Sequenz**

(30) Priorität: 18.05.1994 DE 4417353
(71) Anmelder: BAYER AG, 51368 Leverkusen (DE)
(72) Erfinder: Apeler, Heiner, Dr., D-42115 Wuppertal (DE); Beunink, Jürgen, Dr., D-42329 Wuppertal (DE); Dörschug, Michael, Dr., D-42579 Heiligenhaus (DE); Gottschalk, Uwe, Dr., D-42555 Velbert (DE)

(57) **Zusammenfassung**

Die Erfindung betrifft ein Verfahren zur Herstellung von rekombinantem Aprotinin und rekombinanten Aprotinin Varianten mit der natürlichen N-terminalen Sequenz, wobei das rekombinante Aprotinin bzw. die rekombinanten Aprotinin Varianten als homogen prozessierte, sezernierte Proteine vorliegen.

SEQUENZPROTOKOLL

(1) ALGEMEINE INFORMATION:

(i) ANMELDER:
(A) NAME: Bayer AG
(B) STRASSE: Bayerwerk
(C) ORT: Leverkusen
(E) LAND: Germany
(F) POSTLEITZAHL: D-51519
(G) TELEPHON: 0214/3061455
(H) TELEFAX: 0214/303482

(ii) ANMELDETITEL: Verfahren zur Herstellung von recombinantem
Aprotinin und recombinanten Aprotinin Varianten mit
natuerlicher N-terminaler Sequenz

(iii) ANZAHL DER SEQUENZEN: 4

(iv) COMPUTER-LESBARE FORM:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.25 (EPA)

(2) INFORMATION ZU SEQ ID NO: 1:

(i) SEQUENZ CHARAKTERISTIKA:
(A) LÄNGE: 42 Basenpaare
(B) ART: Nukleinsäure
(C) STRANGFORM: Einzel
(D) TOPOLOGIE: linear

(ii) ART DES MOLEKÜLS: DNS (genomisch)

(iii) HYPOTHETISCH: NEIN

(iii) ANTISENSE: NEIN

(xi) SEQUENZBESCHREIBUNG: SEQ ID NO: 1:

GCAGCATCCT CCGCATTAGC TCGTCCGGAC TTCTGCCTCG AG 42

(2) INFORMATION ZU SEQ ID NO: 2:

(i) SEQUENZ CHARAKTERISTIKA:
(A) LÄNGE: 234 Basenpaare
(B) ART: Nukleinsäure
(C) STRANGFORM: Einzel
(D) TOPOLOGIE: linear

(ii) ART DES MOLEKÜLS: DNS (genomisch)

(iii) HYPOTHETISCH: NEIN

(iii) ANTISENSE: NEIN

(vi) URSPRÜNLICHE HERKUNFT:
(A) ORGANISMUS: Saccharomyces cerevisiae und Rind

(xi) SEQUENZBESCHREIBUNG: SEQ ID NO: 2:

ATGAGATTTC CTTCAATTTT TACTGCAGTT TTATTCGCAG CATCCTCCGC ATTAGCTCGT 60

CCGGACTTCT GCCTCGAGCC GCCGTACACT GGGCCCTGCA GAGCTCGTAT CATCCGTTAC 120

TTCTACAATG CAAAGGCAGG CCTGTGTCAG ACCTTCGTAT ACGGCGGCTG CAGAGCTAAG 180

CGTAACAACT TCAAATCCGC GGAAGACTGC ATGCGTACTT GCGGTGGTGC TTAG 234

(2) INFORMATION ZU SEQ ID NO: 3:

(i) SEQUENZ CHARAKTERISTIKA:
(A) LÄNGE: 77 Aminosäuren
(B) ART: Aminosäure
(C) STRANGFORM: Einzel
(D) TOPOLOGIE: linear

(ii) ART DES MOLEKÜLS: Protein

(iii) HYPOTHETISCH: NEIN

(v) ART DES FRAGMENTS: N-Terminus

(xi) SEQUENZBESCHREIBUNG: SEQ ID NO: 3:

Met Arg Phe Pro Ser Ile Phe Thr Ala Val Leu Phe Ala Ala Ser Ser
1               5                   10                  15

Ala Leu Ala Arg Pro Asp Phe Cys Leu Glu Pro Pro Tyr Thr Gly Pro
            20                  25                  30

Cys Arg Ala Arg Ile Ile Arg Tyr Phe Tyr Asn Ala Lys Ala Gly Leu
        35                  40                  45

Cys Gln Thr Phe Val Tyr Gly Gly Cys Arg Ala Lys Arg Asn Asn Phe
    50                  55                  60

Lys Ser Ala Glu Asp Cys Met Arg Thr Cys Gly Gly Ala
65                  70                  75

(2) INFORMATION ZU SEQ ID NO: 4:

(i) SEQUENZ CHARAKTERISTIKA:
(A) LÄNGE: 19 Aminosäuren
(B) ART: Aminosäure
(C) STRANGFORM: Einzel
(D) TOPOLOGIE: linear

(ii) ART DES MOLEKÜLS: Peptid

(iii) HYPOTHETISCH: NEIN

(v) ART DES FRAGMENTS: N-Terminus

(xi) SEQUENZBESCHREIBUNG: SEQ ID NO: 4:

Arg Pro Asp Phe Cys Leu Glu Pro Pro Tyr Thr Gly Pro Cys Arg Ala
1               5                   10                  15

Arg Ile Ile

## Beschreibung

Die Erfindung betrifft ein Verfahren zur Herstellung von rekombinantem Aprotinin und rekombinanten Aprotinin Varianten mit der natürlichen N-terminalen Sequenz, wobei das rekombinante Aprotinin bzw. die rekombinanten Aprotinin Varianten als homogen prozessierte, sezernierte Proteine vorliegen.

Aprotinin, das auch als "boviner pankreatischer Trypsin-Inhibitor" (BPTI) bezeichnet wird, gehört zur Familie der Inhibitoren vom Kunitz-Typ. Das Spektrum der inhibierbaren Serin-Proteasen umfaßt z.B. Trypsin, Chymotrypsin, Plasmin und Plasma Kallikrein (W. Gebhard, H. Tschesche und H.Fritz, Proteinase Inhibitors, Barrett and Salvesen (eds.), Elsevier Science Publ. BV 375 - 387, 1986).

Aprotinin besteht aus 58 Aminosäuren. Die 3-dimensionale Struktur des Proteins wurde mit Hilfe von Röntgenstrukturanalyse und NMR-Spektroskopie aufgeklärt (Wlodawer et al., J. Mol. Biol. 198 (3), 469 - 480, 1987; Wagner et al., J. Mol. Biol. 196 (1), 227 - 231, 1987; Berndt et al., Biochemistry 32 (17), 4564 - 4570, 1993).

Unter dem Handelsnamen Trasylol wird natürliches Aprotinin für die Behandlung von Pankreatitis eingesetzt. Daneben wird Trasylol seit einiger Zeit in der offenen Herzchirurgie verwendet, nachdem klinische Studien gezeigt hatten, daß eine Behandlung mit Aprotinin den Blutverlust bei derartigen Operationen signifikant verringert und zur Reduktion von Nachblutungen führt (Bistrup et al., Lancet 1, 366 - 367, 1988).

Aprotinin Varianten mit einer veränderten Aminosäure in Position 15 zeigen eine starke inhibitorische Aktivität gegenüber den Elastasen aus Pankreas und Leukozyten und der Protease Cathepsin G (Tschesche et al., U.S. Patent 4,595,674).

Aprotinin Varianten mit der Aminosäure Arginin in Position 15 zeichnen sich im Vergleich zu natürlichem Aprotinin durch eine höhere Affinität gegenüber Plasma Kallikrein aus.

Die Expression rekombinanter Aprotinine wurde beschrieben für E. coli K 12 (Auerswald et al., Biol. Chem. Hoppe Seyler 369, 27 - 35, 1988) und Hefe (EP 0 419 878).

Im Hefesystem wurden Signalsequenzen sekretorischer Hefeproteine, wie z.B. die Signalsequenz des alpha-mating Faktors, mit dem N-Terminus der Aprotinin Varianten verknüpft. Die Prozessierung der prepro-alpha-Faktor-Aprotinin Fusionsproteine erfordert die Aktivität von zwei unterschiedlichen proteolytischen Enzymen. Die sog. Signalpeptidase, die im Endoplasmatischen Retikulum lokalisiert ist, spaltet zunächst das Fusionsprotein zwischen den Aminosäuren 19 und 20. Das hierbei entstehende - in der pro-Region glykosylierte - pro-alpha-Faktor-Aprotinin wird anschließend von einer Endoproteinase gespalten, die spezifisch das dibasische Aminosäurepaar Lys-Arg erkennt. Diese Endoproteinase ist das Produkt des KexII Gens. Die Spaltung der KexII Protease führt somit zur Freisetzung und Sekretion des Aprotinins (A. J. Brake, Methods in Enzymology 185, 408 - 421, 1990; Das et al., Biotechn. Progress 3, 43 - 48, 1987).

Im Fall der rekombinanten Aprotinin und Aprotinin Varianten stellte sich jedoch heraus, daß die KexII Protease nicht in der Lage war, Aprotinine mit der natürlichen N-terminalen Sequenz "Arg-Pro-Asp" abzuspalten. Ein einheitlich und korrekt prozessiertes sezerniertes Aprotinin konnte nicht exprimiert werden.

Durch eine Veränderung der N-terminalen Sequenz des Aprotinins in Form einer Deletion der Aminosäure Prolin in Position 2 bzw. einer Insertion der Aminosäuren Alanin und Glutamin in die Positionen -2 und -1 konnte dieses Prozessierungsproblem gelöst werden (EP 0 419 878). Die aus dieser Modifikation resultierenden Aprotinine waren zu einem hohen Prozentsatz korrekt prozessiert.

Die Veränderung der N-terminalen Sequenz des Aprotinins bzw. der Aprotinin Varianten zum Zweck einer korrekten Prozessierung im Hefesystem ist aber nicht wünschenswert, da die jeweiligen N-terminal modifizierten Aprotinine eine Immunantwort auslösen könnten.

Aufgabe der Erfindung ist es daher, ein Verfahren zur Herstellung von rekombinantem Aprotinin bzw. rekombinanten Aprotinin Varianten mit der natürlichen N-terminalen Sequenz "Arg-Pro-Asp" zur Verfügung zu stellen, wobei das rekombinante Aprotinin bzw. die rekombinanten Aprotinin Varianten als homogen prozessierte, sezernierte Proteine vorliegen.

Die Aufgabe konnte überraschenderweise durch eine vollständige Deletion der alpha-Faktor-pro-Sequenz aus dem prepro-alpha-Faktor-Aprotinin Fusionskonstrukt gelöst werden. Hierbei wird somit die alpha-Faktor-pre-Sequenz direkt mit der Aprotinin Sequenz verknüpft. Für die Prozessierung dieses Konstrukts ist ausschließlich die Signalpeptidase erforderlich. Das rekombinante Aprotinin bzw. die rekombinanten Aprotinin Varianten werden zu einem sehr hohen Prozentsatz korrekt prozessiert und ins Medium sezerniert. Der "Klassische" Weg der Sekretion von Proteinen im Hefesystem, der die Aktivität der KexII Protease erfordert, spielt bei der Verwendung von Konstrukten mit deletierter alpha-Faktor-pro-Sequenz, die in dieser Erfindung beschrieben sind, keine Rolle.

In der WO 89/01968 wurde ebenfalls ausgehend von der Signalsequenz des alpha-mating Faktors ein Verfahren beschrieben, das die Expression von Aprotinin mit einem natürlichen N-Terminus im Hefesystem erlaubt. Da dieses Verfahren aber offensichtlich auf der proteolytischen Aktivität der KexII-Protease beruht, scheint es für die Herstellung von rekombinanten Aprotinin und Aprotinin Varianten mit einer korrekt prozessierten, natürlichen N-terminalen Sequenz ungeeignet zu sein.

Nachstehend werden die im Verlauf der Klonierung, Fermentation und Reinigung des rekombinanten Aprotinins bzw. der rekombinanten Aprotinin Varianten mit der natürlichen N-terminalen Sequenz eingesetzten Methoden aufgeführt.

### Methoden

### Enzyme

Die verwendeten Enzyme (Restriktionsendonucleasen, Alkalische Phosphatase aus Kälberdarm, T4 Polynucleotidkinase und T4 DNA Ligase) wurden von den Firmen Boehringer Mannheim und GIBCO-BRL bezogen und nach Vorschriften der Hersteller eingesetzt.

### Molekularbiologische Techniken

Routinemäßige Klonierungsarbeiten, wie z.B. die Isolierung von Plasmid-DNA aus E. coli (sog. miniprep) und die Transformation von E. coli mit Plasmid-DNA wurden nach Sambrook et al. (Molecular cloning, Cold Spring Harbor, 1989) durchgeführt. Als Wirtsorganismus für Transformationen wurde hauptsächlich der E. coli Stamm SURE® (Stratagene) eingesetzt. Zur Isolierung größerer Mengen Plasmid-DNA wurden Qiagentips (Diagen) verwendet. Die Extraktion von DNA-Fragmenten aus Agarosegelen wurde mit Hilfe von Jetsorb nach Angaben des Herstellers (Genomed) durchgeführt.

Oligonukleotide für "site directed mutagenesis" (Deletion der alpha-Faktor-pro-Sequenz) und Primer für die Sequenzierung wurden mit dem "380 A DNA synthesizer" der Firma Applied Biosystems hergestellt. Die Mutageneseversuche wurden nach einem Verfahren von Eckstein durchgeführt (Taylor et al., Nucl. Acids Res. 13, 8764 - 8785, 1985) unter Verwendungt eines Kits der Firma Amersham-Buchler ("Oligonucleotide-directed in vitro mutagenesis System version 2.1"). Alle Vektorkonstruktionen und Mutageneseexperimente wurden durch Einzel- oder Doppelstrang DNA-Sequenzierung bestätigt. Hierzu wurde ein Kit der Firma US Biochemical Corporation eingesetzt ("Sequenase version 2.0").

### Transformation von Saccharomyces cerevisiae

Hefezellen, z.B. der Stamm SC125A (MATa, ura3-52, suc2) wurden in 10 ml YEPD (2 % Glucose; 2 % Pepton; 1 % Difco Hefeextrakt) angezogen und bei einer O.D. ₆₀₀ₙₘ von 0,6 bis 0,8 geerntet. Die Zellen wurden mit 5 ml Lösung A (1 M Sorbitol; 10 mM Bicin pH 8,35; 3 % Ethylenglycol) gewaschen, in 0,2 ml Lösung A resuspendiert und bei -70°C gelagert.

Plasmid DNA (5 µg) und Carrier DNA (50 µg DNA aus Herringssperma) wurden zu den gefrorenen Zellen gegeben. Die Zellen wurden anschließend durch Schütteln für 5 min bei 37°C aufgetaut. Nach Zugabe von 1,5 ml Lösung B (40 % PEG 1000; 200 mM Bicin pH 8,35) wurden die Zellen für 60 min bei 30°C inkubiert, mit 1,5 ml Lösung C (0,15 M NaCl; 10 mM Bicin pH 8,35) gewaschen und in 100 µl Lösung C resuspendiert. Die Ausplattierung erfolgte auf einem Selektivmedium mit 2 % Agar. Transformanden wurden nach einer Inkubation von 3 Tagen bei 30°C erhalten.

### Fermentation der Hefezellen

### Nährlösungen

Zur Fermentation von Hefezellen zur Expression von Aprotinin oder Aprotinin Varianten mit korrekter N-terminaler Aminosäuresequenz wurden die folgenden Nährlösungen verwendet:

| | Nährlösung | |
|---|---|---|
| Einsatzstoff | SD2 | Sc6 |
| Bacto Yeast Nitrogen Base | 6.7 g/l | - |
| Difco Hefeextrakt | - | 20.0 g/l |
| Glucose | 20.0g/l | 5.0g/l |
| KH₂PO₄ | 6.7g/l | 1.4g/l |
| (NH₄)₂SO₄ | - | 2.0 g/l |
| MgSO₄ x 7 H₂O | - | 0.5 g/l |
| Antischaummittel PPG 2000 | - | 0.1 g/l |

Die Einsatzstoffe wurden in demineralisiertem Wasser angesetzt und der pH-Wert auf pH 5,5 eingestellt. Die Sterilisation erfolgte für 20 min bei 121°C. Glucose wurde in 1/5 des erforderlichen Volumens in demineralisiertem Wasser gelöst, getrennt sterilisiert und nach dem Abkühlen zur übrigen Nährlösung gegeben.

### Stammkonserven

Stammkonserven aller Hefetransformanden wurden durch Abfüllung von 2-ml-Aliquots einer Vorkultur und Lagerung in flüssigem Stickstoff angelegt.

### Vorkulturen

Die Vorkulturfermentationen wurden in 1-Ltr.-Schüttelkolben, gefüllt mit 200 ml SD2-Nährlösung durchgeführt. Die Beimpfung erfolgte mit einer Stammkonserve oder mit einer Einzelkolonie von einer SD2-Agarplatte. Die Kulturen wurden unter ständigem Schütteln für 2 bis 3 Tage bei 26 bis 30°C inkubiert.

### Hauptkulturfermentationen

Die Hauptkulturfermentationen wurden unter Verwendung von 10-Ltr.-Rührkesselfermentern in Sc6-Nährlösung durchgeführt. Die Beimpfung erfolgte mit 3 bis 5 Vol-% einer Vorkultur, wobei vor der Beimpfung die Biomasse aus der Vorkultur abzentrifugiert und in Sc6-Medium resuspendiert wurde. Die Fermentationsbedingungen für die 10-Ltr.-Hauptkultur waren wie folgt:

| | |
|---|---|
| Temperatur | 26 - 30°C |
| Rührerdrehzahl | 600 rpm |
| Belüftungsrate | 0,5 vvm |
| pH-Sollwert | 5,5 (Korrektur mit 5 N NaOH und 5 N H₂SO₄) |

Ab einer Fermentationszeit von 5 Stunden wurden die Kulturen kontinuierlich mit Glucose und Hefeextrakt gefüttert. Die Fütterungsrate wurde über den Respiratorischen Quotienten (RQ-Wert) der Kultur geregelt. Der RQ-Sollwert betrug 1,0. Die Fütterlösung hatte folgende Zusammensetzung:

| | |
|---|---|
| Glucose | 500 g/l |
| Difco-Hefeextrakt | 75 g/l |

Die Bestandteile wurden getrennt in demineralisiertem Wasser gelöst und für 20 min bei 121°C sterilisiert. Nach dem Abkühlen wurden beide Lösungen vereinigt.

Bei Verwendung des induzierbaren Ga110-Promotors oder eines Derivats des Ga110-Promotors erfolgte die Induktion durch Wechsel des Kohlenhydrats in der Fütterlösung von Glucose (500 g/l) zu Galaktose (500 g/l). Die weitere Kontrolle der Fütterungsrate erfolgte dann nicht über den RQ-Wert. Die Fütterungsrate wurde manuell auf den doppelten Wert der Fütterungsrate zum Zeitpunkt der Induktion eingestellt. Die Induktion des Ga110-Promotors erfolgte üblicherweise nach etwa 48 Stunden Fermentationsdauer.

### Zellernte

Nach Beendigung der Fermentation (80 bis 120 Stunden) wurde der Fermenterinhalt auf 10 bis 15°C abgekühlt und die Hefezellen durch Standard-Zentrifugationstechniken (z.B. Becherzentrifuge) oder mittels Querstrom-Mikrofiltration (z.B. Filtron-Minisette-System) aus der Kulturbrühe abgetrennt.

Falls erforderlich, wurden die Zellen gewaschen und die Kulturbrühe sterilfiltriert. Bei Sekretion des heterologen Proteins erfolgte die weitere Produktaufarbeitung aus der zellfreien Kulturbrühe; bei intrazellulärer Expression wurde die Biomasse zur Aufarbeitung verwendet.

### Analytische Methoden

N-terminale Sequenzbestimmungen, Aminosäureanalysen, sowie die Elastase und Trypsin Inhibitionsteste wurden wie in der US-PS 5 164 482 beschrieben durchgeführt.

Die Erfindung wird durch die folgenden Beispiele, Abbildungen und Sequenzinformationen näher erläutert:
- Fig. 1: zeigt eine Restriktionskarte des E. coli/Hefe Schaukelvektors pA228. Die essentiellen Elemente des Vektors sind dargestellt.
- Fig. 2: zeigt die entscheidenden Sequenzen (Nukleotide und Aminosäuren) des prepro-alpha-Faktor-Aprotinin Fusionskonstrukts vor und nach der Deletion der alpha-Faktor-pro-Sequenz. Die Erkennungsstelle für die Restriktionsendonuklease HindIII, die Schnittstelle für die Signalpeptidase und die KexII Protease sind ebenfalls eingezeichnet.

### Beispiele

### Beispiel 1

### Herstellung eines Hefe-Expressionsvektors zur Sekretion von rekombinantem Arg-15-Aprotinin mit der natürlichen N-terminalen Sequenz "Arg-Pro-Asp"

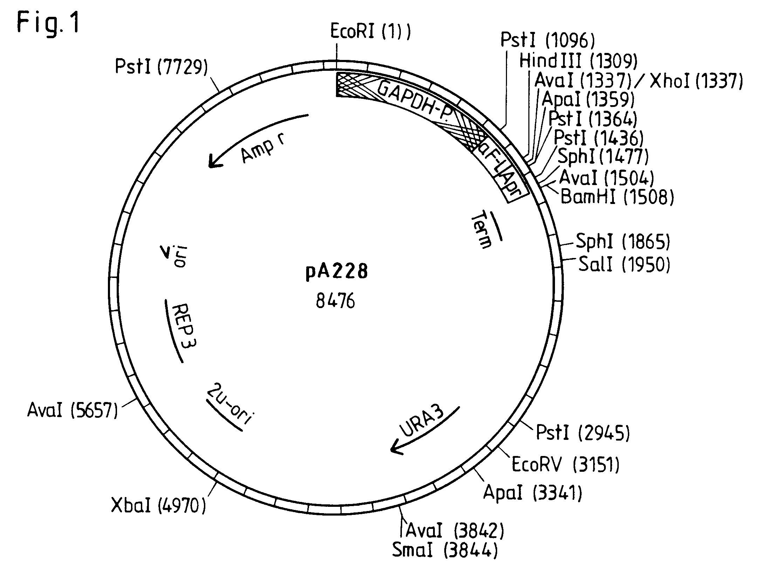

Der E. coli/Hefe Schaukelvektor pA228 (Fig. 1) wurde als Ausgangsmaterial für die Konstruktion eines Hefe-Sekretionsvektors eingesetzt, in dem die Arg-15-Aprotinin-Sequenz direkt mit der alpha-Faktor-pre-Sequenz verknüpft ist.

Der Vektor pA228 trägt ein Ampicillin Resistenzgen (bla) und ein URA3 Gen als selektierbare Markergene für E. coli und Hefe. Weitere essentielle Elemente des Vektors sind der Col E1- und der 2µ Ursprungspunkt der Replikation (ori). Der REP3 Locus befindet sich ebenfalls in dieser Region. Ein 1300 Bp großes EcoRI-HindIII-Fragment trägt den GAPDH Promotor und die N-terminale pre-pro-Sequenz des Hefe alpha-Faktor-Vorläuferproteins (Kurjan und Herskowitz, Cell 30, 933 - 943, 1982). Durch Einführung einer modifizierten Arg-15-Aprotinin cDNA als HindIII-BamHI Fragment wurde die Erkennungsstelle für die KexII Protease ("Lys-Arg") innerhalb der alpha-Faktor-pre-pro-Sequenz wieder hergestellt (EP 0 419 878).

Am 3'-Ende der Arg-15-Aprotinin Sequenz trägt der Vektor ein BamHI-SalI Fragment des Hefe URA3 Gens, daß in dieser Position als Terminationssignal für die Transkription fungiert (Yarger et al., Mol. Cell. Biol. 6, 1095 - 1101, 1986).

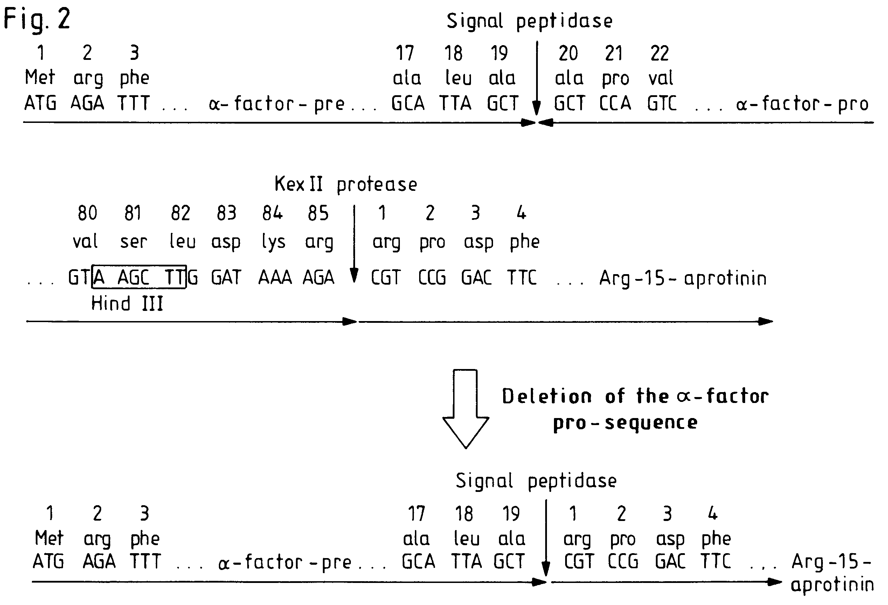

Der Vektor pA228 wurde mit den Restriktionsendonukleasen EcoRI und BamHI geschnitten. Das hierbei entstehende 1500 Bp große DNA Fragment, das die Sequenzinformationen trägt für den GAPDH Promotor, die alpha-Faktor-pre-pro-Sequenz und das Arg-15-Aprotinin Gen, wurde in den ebenfalls mit EcoRI und BamHI geschnittenen Vektor M13mp19 umkloniert. Einzelsträngige DNA wurde hergestellt und einer Deletions-Mutagenese mit folgendem Oligonukleotid unterzogen:
5' GCAGCATCCTCCGCATTAGCTCGTCCGGACTTCTGCCTCGAG 3'.
Das "Screening" der Plaques erfolgte durch eine Restriktionsanalyse ausgehend von doppelsträngiger M13 RF DNA (Replikative Form). Positive Klone konnten durch einen Restriktionsverdau mit dem Enzym HindIII identifiziert werden, da durch die Deletion der alpha-Faktor-pro-Sequenz eine HindIII Schnittstelle verschwindet. Die Deletion der alpha-Faktor-pro-Sequenz und das korrekte Verknüpfen der alpha-Faktor-pre-Sequenz mit dem Arg-15-Aprotinin Gen wurden anschließend durch Sequenzierung bestätigt (Fig. 2). Ein 1300 Bp großes DNA Fragment wurde mit EcoRI und BamHI aus einem ausgewählten M13mp19 Klon herausgeschnitten, über Agarosegel-Elektrophorese gereinigt und in einen ebenfalls mit EcoRI und BamHI geschnittenen und gelgereinigten Vektor pA228 kloniert. Hefezellen (SC125A) wurden mit dem aus dieser Klonierung resultierenden Vektor pAP02 transformiert.

Andere E. coli/Hefe Schaukelvektoren mit unterschiedlichen Promotoren, wie z.B. der konstitutive MFα1 oder der induzierbare GAL 10 Promotor, können in ähnlicher Weise hergestellt werden und führen ebenfalls zur Sekretion von Aprotinin und Aprotinin Varianten mit einem natürlichen N-Terminus.

Daneben ist es selbstverständlich auch möglich, Schaukelvektoren mit anderen Hefe-Replikationsursprüngen, wie z.B. das chromosomal autonom replizierende Segment (ars), einzusetzen.

Geeignete selektierbare Markergene sind neben dem URA3 Gen solche Gene, die einer auxotrophen Hefemutante zur Prototrophie verhelfen, wie z.B. die LEU2, HIS3 oder TRP1 Gene. Außerdem können selbstverständlich auch Gene eingesetzt werden, deren Produkte Resistenz gegenüber verschiedenen Antibiotika, wie z.B. dem Aminoglykosid G418, vermitteln.

Die Expression von Aprotinin und Aprotinin-Varianten mit der natürlichen N-terminalen Sequenz ist nicht nur in der Hefe Saccharomyces cerevisiae möglich. Andere Hefen, wie z.B. Schizosaccharomyces pombe oder die methylotrophen Hefen Pichia pastoris und Hansenula polymorpha sind ebenfalls hierfür geeignet.

Neben der pre-alpha-Faktor Signalsequenz können auch andere Signalsequenzen, wie z.B. die Signalsequenzen der Phosphatase (PHO1 und PHO5) und der Invertase verwendet werden, soweit für die Prozessierung ausschließlich die Signalpeptidase erforderlich ist.

### Beispiel 2

### Expression von Aprotinin und Aprotinin Varianten mit natürlicher N-terminaler Aminosäuresequenz unter Verwendung konstitutiver Promotoren

Hefetransformanden mit dem pAP02-Vektor oder einem Analogon dieses Vektors mit einem konstitutiven Promotor (z.B. alpha-Mating Faktor (MFα1)-Promotor, GAPDH-Promotor, TPI-Promotor) wurden im 10-Ltr.-Maßstab bei 28°C kultiviert. Während der Fermentation erfolgte die Produktquantifizierung unter Verwendung des Elastase-Inhibitionstests (im Falle der Espression von Val15-Aprotinin, Val15-Leu17-Aprotinin oder Val15-Leu17-Arg19-Aprotinin) bzw. unter Verwendung des Trypsin-Hemmtests (im Falle der Espression von Aprotinin, Arg15-Aprotinin oder Arg15-Ala17-Aprotinin). Die Fermentationsdauer betrug 96 Stunden. Die bei Fermentationsende erzielte Biomassekonzentration lag bei 31 g/l TG. Die Produktkonzentration betrug etwa 10 mg/l. Nach Abtrennung der Zellen durch Zentrifugation (15 min, 6.500 x g, 4°C) und Sterilfiltration erfolgte die Produktaufarbeitung aus der zellfreien Kulturbrühe.

### Beispiel 3

### Expression von Aprotinin und Aprotinin Varianten mit natürlicher N-terminaler Aminosäuresequenz unter Verwendung induzierbarer Promotoren

Hefetransformanden mit einem Analogon des pAP02-Vektors mit einem induzierbaren Promotor (z.B. Gal10-Promotor oder ein Derivat des Gal10-Promotors) wurden im 10-Ltr.-Maßstab bei 28°C kultiviert. Nach einer Fermentationsdauer von 48 Stunden erfolgte die Induktion durch Wechsel des in der Fütterlösung verwendeten Kohlenhydrats von Glucose nach Galaktose. Während der Fermentation erfolgte die Produktquantifizierung unter Verwendung des Elastase-Inhibitionstests (im Falle der Expression von Val15-Aprotinin, Val15-Leu17-Aprotinin oder Val15-Leu17-Arg19-Aprotinin) bzw. unter Verwendung des Trypsin-Hemmtests (im Falle der Expression von Aprotinin, Arg 15-Aprotinin oder Arg15-Ala17-Aprotinin). Die Fermentationsdauer betrug 96 Stunden. Die bei Fermentationsende erzielte Biomassekonzentration betrug 24 g/l TG; die erzielte Produktkonzentration betrug etwa 15 mg/l. Nach Abtrennung der Zellen und Sterilfiltration erfolgte die Produktaufarbeitung aus der zellfreien Kulturbrühe.

Analog zu diesem Verfahren können auch andere induzierbare Promotoren zur Expression von Aprotinin oder Aprotinin Varianten mit natürlicher N-terminaler Aminosäuresequenz eingesetzt werden. In Abhängigkeit von der Art des gewählten Promotors muß eine geeignete Induktionstechnik eingesetzt werden.

### Beispiel 4

### Reinigung einer rekombinanten Aprotinin Variante

5 Liter Fermentationsüberstand wurden mittels Zugabe von 150 ml Citronensäure auf pH 3.0 eingestellt. 200 ml SP-Sepharose-FF Gel (Pharmacia), äquilibriert mit 50 mM Natriumcitrat, pH 3.0, wurden zugegeben und der gesamte Ansatz 45 Minuten bei Raumtemperatur gerührt. Das Gel wurde mit 50 mM Natriumcitrat, pH 3.0, gewaschen und in eine Säule gefüllt. Die Säule wurde nacheinander mit 50 mM Natriumcitrat, pH 3.0, 50 mM TRIS, pH 9.0 und 20 mM HEPES, pH 6.0, gewaschen. Das gebundene Material wurde batchweise mit 0,25 M, 0,55 M und 1 M NaCl in 20 mM HEPES, pH 6.0, eluiert. Fraktionen, in denen Trypsininhibitoraktivität gefunden wurde, wurden vereinigt und mit Citronensäure auf pH 3.0 eingestellt. Zur Verringerung der Leitfähigkeit wurde Wasser hinzugegeben (Endwert ≦ 17,5 mS/cm). Anschließend wurde das Material auf eine mit 50 mM Natriumcitrat, pH 3.0 äquilibrierte S-Sepharose-HP Säule (Pharmacia, 100 ml) aufgetragen. Das Produkt wurde mit einem linearen NaCl-Gradienten (0-1 M NaCl in 20 mM HEPES, pH 6,0) eluiert. Fraktionen, in denen Trypsininhibitoraktivität gefunden wurde, wurden vereinigt, gegen 50 mM NH₄HCO₃ dialysiert und anschließend lyophilisiert.

### Beispiel 5

### Charakterisierung der isolierten Aprotinin Variante

Das lyophilisierte Material wurde mittels einer Bestimmung der N-terminalen Aminosäuren (Fig. 3) und einer Analyse der Aminosäurezusammensetzung (Fig. 4) charakterisiert. Neben der Aprotinin Variante mit dem korrekt prozessierten N-Terminus waren keine weiteren Aprotininsequenzen nachweisbar.

**Tabelle**

| Aminosäurezusammensetzung einer gereinigten Arg-15-Aprotinin Variante mit dem korrekt prozessierten, natürlichen N-Terminus | | |
|---|---|---|
| Aminosäure | Theoretischer Wert | Faktorisierte Aminosäure |
| CYS | 6 | 4.83 |
| ASP | 5 | 5.00 |
| THR | 3 | 2.72 |
| SER | 1 | 1.02 |
| GLU | 3 | 3.30 |
| GLY | 6 | 6.14 |
| ALA | 6 | 5.99 |
| VAL | 1 | 0.90 |
| MET | 1 | 0.72 |
| ILE | 2 | 1.43 |
| LEU | 2 | 1.83 |
| TYR | 4 | 3.71 |
| PHE | 4 | 4.06 |
| HIS | - | 0.12 |
| LYS | 3 | 3.61 |
| ARG | 7 | 7.02 |
| PRO | 4 | 3.70 |
| TRP | - | n.d. |

## Patentansprüche

1. Vektor zur Expression von Aprotinin und von Aprotinin Varianten mit korrektem N-Terminus, enthaltend
a) das Gen für das Aprotinin oder die Variante und
b) als Leader direkt vor diesem Gen die Nukleotidsequenz für eine pre-Sequenz deren Prozessierung durch die Signalpeptidase erfolgt.

2. Vektor nach Anspruch 1, wobei der Leader die Signalsequenz vom pre-alpha-Faktor, PHO1, PHO5 oder von der Invertase stammt.

3. Verfahren zur Herstellung von Aprotinin und Aprotinin Varianten mit korrektem N-Terminus durch Kultivieren von Mikroorganismen und Isolieren des Aprotinins bzw. der Variante aus dem Kulturüberstand, dadurch gekennzeichnet, daß der Mikroorganismus den Vektor nach Anspruch 1 oder 2 enthält.
